# EUROPEAN PATENT APPLICATION

(11) **EP 3 785 719 A1**
(43) Date of publication of application: **03.03.2021**
(21) Application number: 19193982.6
(22) Date of filing: 28.08.2019
(51) Int. Cl.: A61K 31/7084, A61P 37/04

(54) **NEW USE OF CYCLIC DINUCLEOTIDES**

(71) Applicant: Helmholtz-Zentrum für Infektionsforschung GmbH, 38124 Braunschweig (DE)
(72) Inventor: GUZMAN, Carlos A., 38124 Braunschweig (DE); LIRUSSI, Dario, 37073 Göttingen (DE); EBENSEN, Thomas, 30853 Langenhagen (DE); WEISSMANN, Sebastian, 38106 Braunschweig (DE)
(74) Representative: Gramm, Lins & Partner Patent- und Rechtsanwälte PartGmbB

(57) **Abstract**

In a first aspect, the present invention relates to cyclic dinucleotide compounds, in particular, c-di AMP for use in a method of inducing or promoting an immune response in an individual wherein said individual is a neonate or infant. The compound according to the present invention is particularly useful for use in therapeutic or prophylactic vaccination. In a further aspect, pharmaceutical compositions comprising the compound according to the present invention as an adjuvant, a pharmaceutically active ingredient for use as defined herein, in particular, as an adjuvant in a vaccine for unborn children, neonates and infants are provided. Finally, the present invention relates to a kit comprising a compound according to the present invention as an adjuvant, an antigen comprising antigenic structure as active vaccination component, in particular, to the use of said kit in a use of preventing or treating a disease.

## Description

In a first aspect, the present invention relates to cyclic dinucleotide compounds, in particular, c-di AMP for use in a method of inducing or promoting an immune response in an individual wherein said individual is a neonate or infant. The compound according to the present invention is particularly useful for use in therapeutic or prophylactic vaccination. In a further aspect, pharmaceutical compositions comprising the compound according to the present invention as an adjuvant, a pharmaceutically active ingredient for use as defined herein, in particular, as an adjuvant in a vaccine for unborn children, neonates and infants are provided. Finally, the present invention relates to a kit comprising a compound according to the present invention as an adjuvant, an antigen comprising antigenic structure as active vaccination component, in particular, to the use of said kit in a use of preventing or treating a disease.

### Prior art

Immunization in early life is a major public health imperative, but remains a challenging field. The immature immune responses in newborns increase their susceptibility to classic and opportunistic infections, which in turn account for at least 5.5 million neonatal infections every year. Newborns remain indeed particularly vulnerable in the first few months of their life to life threatening infections. In this regard, vaccination of newborns represents a key global strategy to overcome morbidity and mortality due to infection in early life.

The neonatal period of life is a window of opportunity for immunizations, especially because barriers like skin and mucosae are more permeable to vaccine antigens, and the associated microbiota is underdeveloped. Interestingly, it was described recently that neonatal vaccines can also confer protection against vaccine annulated diseases, especially when they are accompanied by breast feeding (Alam, M. J., et al., 2015, Vaccine 33 (1):18-21. doi: 10.1016/j.vaccine.2014.10.075). Nevertheless, due to the high frequency of immature antigen-presenting cells (APCs) and the increased Th2 and Th17 cytokine profiles present in newborns, it was assumed that vaccination during the neonatal and infant life could result in decreased immunity rather than protection of the vaccinated newborn.

It was shown that administration of a prime dose of vaccine during the neonatal or infant life period can decrease the immune response to an immunization boost later in the life, and therefore reduce protection (Carazo Perez, S., et al., 2017, Clin Infect Dis. doi: 10.1093/cid/cix510.). This perception was also supported by failed vaccination trials were the use of adjuvants capable of generating a good antibody immune response but not a cellular cytotoxic response had resulted in the complete failure of the infant Respiratory Syncytial Virus (RSV) vaccine. Further, the use of alum-based adjuvants has been reported to increase the risk for potential adverse reactions in newborns, (Shaw, C. A., and L. Tomljenovic. 2013, Immunol Res 56 (2-3):304-16. doi: 10.1007/s12026-013-8403-1). It was also shown that the generation of cytotoxic T Lymphocytes (CTL) by these adjuvants is absent or very poor. In this regard, CTL responses were in turn shown to be able to inhibit RSV vaccine-enhanced disease (Olson, M. R., and S. M. Varga. 2007, J Immunol 179 (8):5415-24) of note, the CTL is one of the few immune response features that are functional in a neonates and can be enhanced by vaccination (see e.g. Hermann, E., et al., 2002, Blood 100 (6):2153-8). Therefore, the availability of adjuvants promoting Th1 and CTL responses meanwhile boosting the antibody production would represent a valuable asset for the development of a neonatal vaccines.

With respect to influence vaccines, newborn and infant vaccination is effected by administration of two doses to infant populations of age 6 to 48 months (Grohskopf, L. A., et. al., 2017. MMWR Recomm Rep 66 (2):1-20. doi: 10.15585/mmwr.rr6602al), meanwhile the same vaccine is applied only once to adults. This lack of immunogenicity in infants is in part due to the mistargeting or a lack of adjuvantation in a vaccine that was produced based on the adult immune system, where humoral responses correlated with protection. The extrapolation from the adult to the child-infant vaccination setting explains in part previous vaccination failures. In fact, parameters and standards are used for adults are not suitable or optimal for the induction and evaluation of early life immunity. This was demonstrated in trials carried out in other high risk populations, such as the elderly as well.

That is, presently vaccine strategies for neonatal and infant vaccination are urgently needed do to reactogenicity and the lack of immunogenicity issues. In particular, there is a need for new adjuvants tailored for neonatal and infant vaccination. This is particularly true since the parameters and standards are used for adults are not suitable or optimal for the induction and evaluation of early life immunity. The immune system of neonates and infants are marked by maternal antibody and pure generation of T-cell and B-cell memory in a neonates and infants, thus, resulting in adequate adaptive immunity from vaccinating this population compare to older children and adults.

At present, the scheme of immunizations for children starts with immunizations against hepatitis B within the first month and continues with vaccination after two months after birth.

Almost all vaccines work through induction of serum or mucosal antibodies, especially in young infants where the lack of previous antigen exposure limits the effectiveness of T-cell responses. Impaired responses of neonatal APC to many stimuli is a key hurdle to overcome in developing infective neonatal vaccines.

At present there is only one hepatitis B vaccine for neonatal administration in the vaccine market. The birth dose of thiomersal-free monovalent hepatitis B vaccine is given to prevent vertical transmission of hepatitis B virus (HBV) from a carrier mother, and also prevent horizontal transmission in the first months of life from a carrier among household or other close contacts with HBV. The birth dose is given intramuscularly when the neonate is physiologically stable, preferably within 24 hours of birth. Adjuvants are important in vaccine development. Adjuvants effected in adults however may not need the specific requirements for activating the early life immune system. In several studies, the neonatal adjuvanticity of different adjuvants including TLR4 (glucopyranosyl lipid adjuvant-squalene emulsion), TLR9 (IC31 and CAF01) agonists, which are all induce germinal centers (GCs) and were able to stimulate potent antibody responses to influenza hemagglutinin (HA) in adult mice. Only the HA/CAF01 elicited significantly higher and sustained humoral responses, engaging neonatal B cells to differentiate into GCs already after a single dose.

For neonates vaccines containing simple aluminum salts have been proved. Recently, complex aluminum salts have been licensed for use in the US and other countries in vaccines used in infants, beginning at six weeks of age, older children and adults. However, these molecules were not tested in neonates or there are not effective for use under neonatal conditions.

Cyclic dinucleotides (CDN) have been described as useful adjuvants in immune modulating immune responses. E.g. EP 1 959 989 B1 described the use of cyclic dinucleotides as adjuvants for therapeutic or prophylactic vaccination. Further, other cyclic dinucleotides are known for more than 10 years suitable as immune modulating components. Also the CDN are described as representing active principals in the treatment of various diseases.

Variants of cyclic di-adenosine monophosphate (CDA) described including thiophosphonate derivatives. For example, a product ADUS 100 of GSK exist, a biphosphorothioate analog of c-di-AMP, also named c-di-AMP (RPRP). In addition, fluorated c-di-AMP are described in the art. Similar compounds are described not only of c-di-AMP but also for c-di-GMP or combinations of c-di-AGMP.

However, a problem of most of the CDN commercially available is the problem of long term maintenance in the body since the compounds are not metabolized naturally. This is not only a problem of the CDN compounds commercially available so far but also for other adjuvants. For example, alum, a very old well known adjuvant remain in the body of vaccinated people since it is encapsulated therein and, thus, alum can be detected in the body of vaccinated people after years.

Accordingly, there is a need for vaccination strategies and compounds allowing vaccinations of unborn children, neonates and infants of suitable compositions promoting an immune response, in particular, suitable adjuvants.

### Brief description of the present invention

In a first aspect, the present invention relates to a compound according to formula (I) 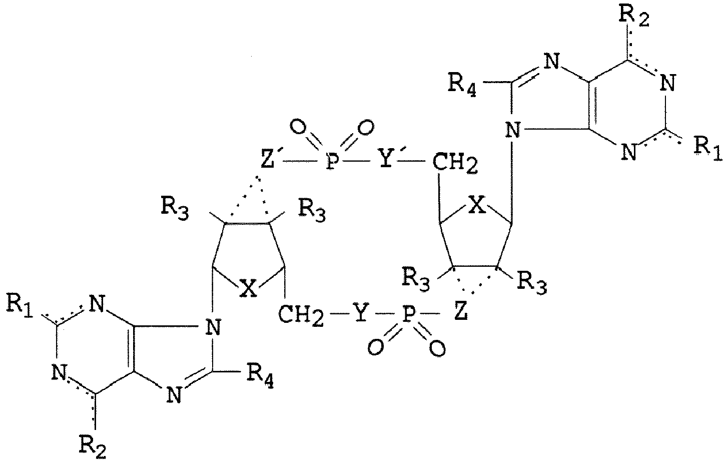 wherein
X is independently from one another S, N, O, CH₂;
Y, Y' is independently from one another NH, CH₂, O;
Z, Z' is independently from one another NH, CH₂, O;
R₁ is independently from one another hydrogen or O or absent;
R₂ is independently from one another NH₂, O, H, or a hydrogen;
R₃ is independently from one another absent if a covalent bond is present between the Z or Z' and the C atom, or is hydrogen, OH, halogen, a straight or branched C₁-C₆ alkyl group, or a straight or branched C₁-C₆ alkoxy group which may optionally be substituted;
R₄ is independently from one another hydrogen, halogen, or a straight or branched C₁-C₆ alkyl group which may optionally be substituted;
... is a single or double bond;
or conjugates thereof, and salts or solvates thereof, for use in a method of inducing or promoting an immune response in an individual wherein said individual is an unborn child, neonate or infant.

In particular, the present invention relates to the use of c-di-AMP (CDA) as an adjuvant in the prophylactic or therapeutic treatment of a disease, in particular, therapeutic or prophylactic vaccination of neonate or infant. In a further aspect, the present invention relates to a pharmaceutical composition comprising the cyclic dinucleotide according to formula I, in particular, CDA. The pharmaceutical composition is particularly useful as a vaccine for unborn children, neonates and infants.

Finally, the present invention comprises a kit and its use, said kit comprise the CDN of formula I according to the present invention as an adjuvant, an antigen comprising antigenic structure as active vaccination component and, optionally, a pharmaceutical accepted carrier, diluent, preservative, adjuvants, other in the compound as defined in the present invention, immunodulators or excipients.

Further, the present inventions describes a method of inducing or promoting an immune response during a prophylactic or therapeutic treatment of a disease or as a therapeutic or prophylactic vaccination. Said method comprises the step of administering the compound of formula I, in particular, CDA as an adjuvant to an individual, namely, an unborn child, neonate or infant, in particular, use of administering the same is an adjuvant in combination with an antigen.

### Brief description of the drawings

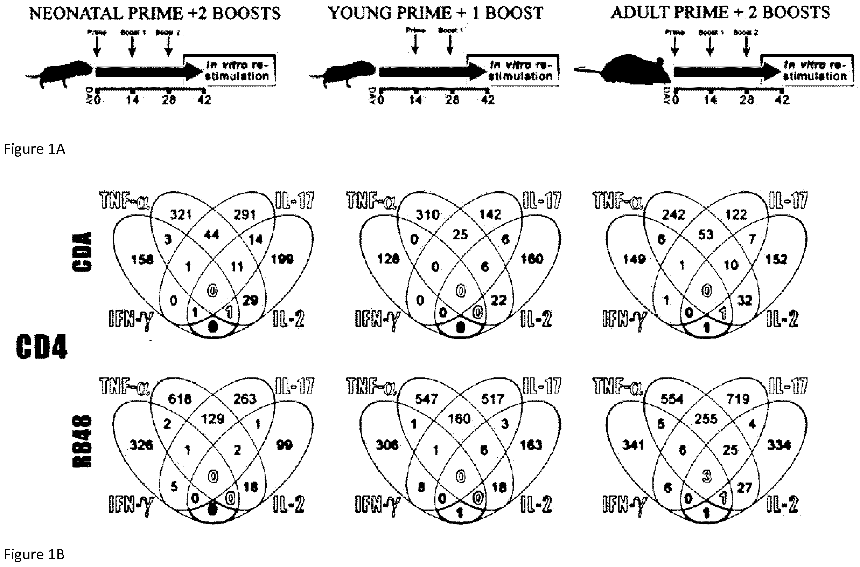

**Figure 1****: Mice antigen specific cytokine responses to neonatal vaccination with different adjuvants.** A) Different vaccination strategies with OVA antigen and adjuvants (R848 and CDA). Neonatal mice received a prime (6-9 days old) followed by two boost doses (left diagram, neonatal prime + 2 boosts). Alternatively, the neonatal prime was skipped (center diagram, young prime + boost) or mice received the three doses as adult (right diagram, adult prime + 2 boosts). B) Venn diagrams depicting number of CD4 T lymphocytes cytokine producers by millions of cells measured by flow cytometry, after in vitro re-stimulation of splenocytes and surface and intracellular cytokine staining. Numbers in white are quadruple and triple cytokine producers regarded as indicators of vaccine efficacy (i.e. producers of IL-2, IFN-γ and TNF-α). Treatments depicted in A) correspond to the diagrams in the column below, where it is R848 or CDA adjuvantation. C) Venn diagrams depicting the number of CD8 T lymphocytes positive for individual or multiple intracellular cytokine staining. White numbers are quadruple or triple cytokine producers by millions of cells analyzed. Results are average of 3-5 mice per group.

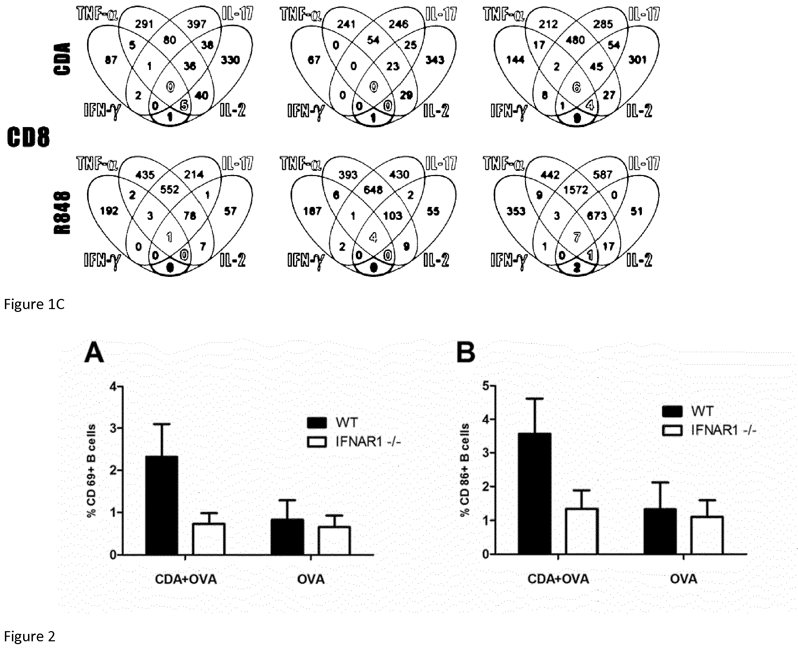

**Figure 2****: Neonatal vaccination with CDA induce B cell activation and maturation.** Percentage of activated B cells after a prime-dose in neonatal mice. A) Frequency of CD69+ B cells or B) CD86+ B cells from WT (B6) or IFNAR1-/- (IFN receptor KO) mice vaccinated with CDA+OVA or OVA alone to determine the dependence of B cell maturation on CDA induced IFN-α/β. Results are from one representative experiment out of 3 independent experiments. Differences are not significant by a non paired student t test (p≤ 0.05).

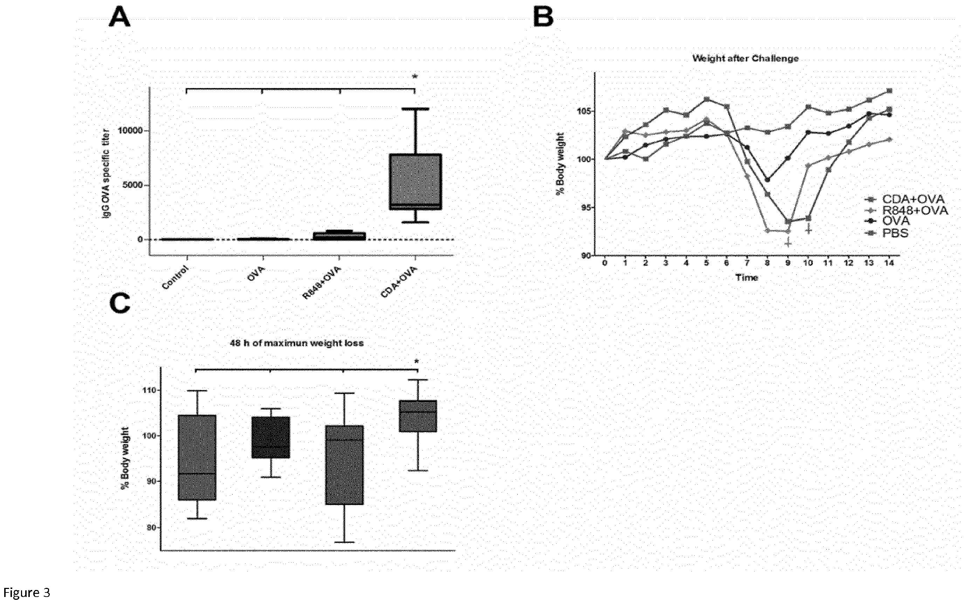

**Figure 3****: Neonatal vaccination with CDA confers protection in adult mice.** Mice intranasally vaccinated with CDA+OVA, R848+OVA, OVA or PBS (control) were challenged by 10⁵ PFU of the H1N1 PR8 influenza A/WSN/33 (WSN)-OVA(I) virus, which expresses the SIINFEKL peptide in the hemagglutinin domain. A) Antigen specific IgG from blood serum was measured by ELISA from blood sampled previous to challenge. Mice Vaccinated with CDA+OVA as neonates displayed significantly higher titers than other vaccinations. B) Weight loss was recorded during 14 days after infection as an inverse correlate of protection, where dead animals were found only in the PBS and the R848 groups (inverted crosses in plot B). C) The differences between groups on the 48 h of maximum weight loss was significant between CDA+OVA vaccinated animals and the other treatments. Differences were significant by a non paired t test (p≤ 0.05). Results are average from 3-5 mice per group, from one representative out of two experiments.

### Detailed description of the present invention

The present invention relates in a first aspect to a compound according to formula (I) 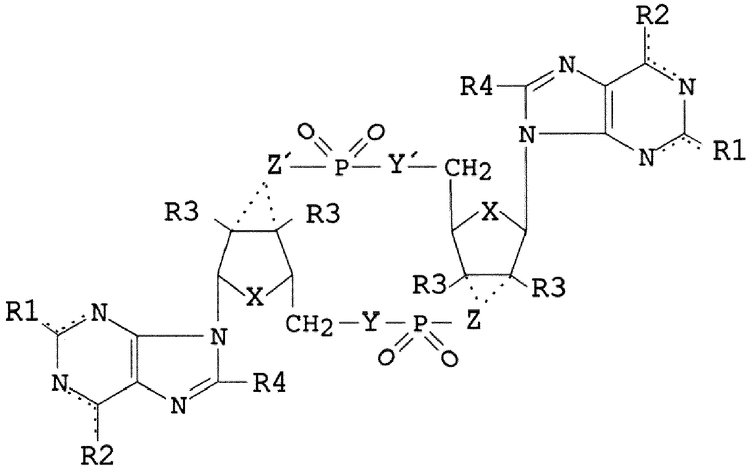 wherein
X is independently from one another S, N, O, CH₂;
Y, Y' is independently from one another NH, CH₂, O;
Z, Z' is independently from one another NH, CH₂, O;
R₁ is independently from one another hydrogen or O or absent;
R₂ is independently from one another NH₂, O, H, or a hydrogen;
R₃ is independently from one another absent if a covalent bond is present between the Z or Z' and the C atom, or is hydrogen, OH, halogen, a straight or branched C₁-C₆ alkyl group, or a straight or branched C₁-C₆ alkoxy group which may optionally be substituted;
R₄ is independently from one another hydrogen, halogen, or a straight or branched C₁-C₆ alkyl group which may optionally be substituted;
... is a single or double bond;
or conjugates thereof, and salts or solvates thereof, for use in a method of inducing or promoting an immune response in an individual wherein said individual is an unborn child, neonate or infant.

As used herein, the term "neonate" refers to newborn individuals. In human, neonate refers to newborn indivuduals from birth to an age of six months.

As used herein, the term" infant" refers to young individuals (children). In humans, infants refer to children aged 6 months to 6 years.

As used herein, the term "unborn child" or "unborn children" refers to individuals not yet born but being in the uterus of their mother.

It is preferred that the individual is a human.

The term "adjuvant" means substances which are added and/or co-formulated in an immunization to the active antigen, i.e. the substance which provokes the desired immune response, in order to enhance or elicit or modulate the humoral and/or cell-mediated (cellular) immune response against the active antigen. Preferably, the adjuvant, as described herein, is able to enhance or elicit the innate immune response.

The term "therapy" or "treatment" refers to a process that is intended to produce a beneficial change in the condition of an individual like a mammal, e.g. a human, often referred to as a patient, or animal. A beneficial change can, for example, include one or more of: restoration of function, reduction of symptoms, limitation or retardation of progression of a disease, disorder, or condition or prevention, limitation or retardation of deterioration of a patient's condition, disease or disorder. Such therapy usually encompasses the administration of drug, among others.

In particular, the therapy or treatment include vaccination of an individual.

As used herein, the term "pegylated" refers to the conjugation of a compound moiety with conjugate moiety (ies) containing at least one polyalkylene unit, in particular, the term pegylated refers to the conjugation of the compound moiety with a conjugate moiety having at least one polyethylene glycol unit.

As used herein, the term "conjugate" refers to compounds comprising a conjugate moiety and a compound moiety. The compound moiety is any one of formula (I). The term "conjugate moiety" refers to a moiety which is linked to the compound according to formula (I). The conjugate moiety aims to increase the applicability of the compounds disclosed herein.

As used herein, the term "antigenic structure" or "antigen" refers to a structure capable of causing a cellular or humoral immune response. The antigenic structure, also known as epitope, is the part of the antigen which is presented by the MHC or MHC like molecules. Further, the epitope or antigenic structure represents the part of an antigen recognized by antibodies directed against said antigen.

As used herein, the term "modulate an immune response", "inducing an immune response" or "promoting an immune response" refers to any change of the present state of the immune response. The immune response may be modulated, induced or promoted in so far that the response is elicited or a pre-existing immune response is enhanced or decreased. In addition, the immune response may be modulated, induced or promoted by shifting the immune response from a more humoral to a more cellular immune response or vice versa.

In addition, the modulation, induction or promoting of the immune response may encompass the activation or enhancement of the innate immune response.

As used herein, the term "individual" or "subject" which is used herein interchangeably refers to a neonate or infant a need of a therapy or prophylaxis. Preferably, the subject is a mammal, particularly preferred a human. The term "animal" does not include the term "human".

As used herein, the term "carrier" refers to a diluent, adjuvant, other than the adjuvant of formula (I) as described herein, excipient or vehicle.

With the term "which may be substituted" is meant the substitution with a straight or branched C1 to C6 alkyl group or a straight or branched C1 to C6 alkoxy group and/or with a halogen, hydroxyl group or carboxyl group.

In an embodiment, the compound according to formula (I) is c-di-AMP. In another embodiment, the compound is a conjugate of the compound of formula (I), namely a conjugate of c-di-AMP with a conjugate moiety being methoxypolyethylenglycol-carbonyl residue.

As used herein, the term "CDN" or "Cyclic dinucleotide" refers to the compounds of formula (I) unless otherwise indicated.

The term "CDA" as used herein refer to cyclic di-AMP or c-di-AMP unless otherwise indicated. In particular, CDA or c-di-AMP refer to the non-fluorinated and/or non-thionated CDA molecule.

The linkage between the phosphate and the sugar moiety may be via the C3 atom (3') or the C2 atom (2') of the sugar residue. That is, the compound according to formula (I) may be a 2', 2'-CDN, a 3', 2'-CDN, a 2', 3'-CDN, or a 3', 3'-CDN, like a 2', 2'- c-di-AMP, a 3', 2'- c-di-AMP, a 2', 3'- c-di-AMP, or a 3', 3'- c-di-AMP. In formula (I) the dotted lines between Z or Z' and the O at position 2 and 3 of the sugar moiety identifies that a covalent bond may be present or absent. Binding is either via the C2 or C3 atom of the sugar moiety. When binding of the phosphate moiety is via the C3 atom, R₃ is absent at C3 and is present at C2. That is, R₃ is present at the C-atom which is not linked with the phosphate moiety.

In an embodiment of the present invention, the compound according to the present invention is for use as an adjuvant in the prophylactic or therapeutic treatment of a disease. In this connection, prophylactic treatment of a disease refers to embodiments of vaccination. That is, the compound according to the present invention is for use in therapeutic or prophylactic vaccination of unborn children, neonates or infants.

The present inventors surprisingly recognize that the compounds according to the present invention are useful in eliciting, inducing or promoting an immune response in the unborn child, neonate or infant. That is, for the first time it is demonstrated that the CDN according to the present invention, namely the compound according to formula (I), in particular the CDA access adjuvant immunogenicity in human unborn children, neonates and infants. It is demonstrated in the examples that CDA has a stimulatory capacity on cells derived from human cord blood as well as the immunogenicity and efficacy of CDA adjuvant formulations in neonatal model superior to R848 representing the gold standard as a neonatal adjuvant.

In particular, the CDN according to formula (I) compared to other known adjuvants, like the c-GAMP, induces substantial immune response in unborn children, neonates and infants. Furthermore, the compounds according to the present invention are known to represent compounds which are metabolized in the human body, thus, do not represent compounds which stress the individual, as it is the case for other adjuvants including alum or fluorinated or thionated CDN.

In a further embodiment, the present invention relates to compounds according to the present invention for use as a mucosal adjuvant, in particular, for intranasal, intra-NALT, oral, intra-rectal, intrafollicular, transfollicular, intrapulmonary, intrabronchial, intratekal, conjungtival, intravaginal or intraurethral administration, administration into the milk ducts of the breast or by inhalation.

Alternatively, the compound is for use according to the present invention for parenteral administration, in particular, for subcutaneous, intravenous, intradermal, intrafollicular or intramuscular administration.

The skilled person is well aware of suitable weight of administration as well as required dosage. This is particularly true for the individuals to be treated according to the present invention, namely, neonates and infants.

In case of unborn children administration is e.g. by the umbilical cord or other suitable ways of administration with the pregnant women.

In an embodiment, the compound is a conjugate of compound of formula I with a conjugate moiety which is a water-soluble and physiologically tolerated polymer.

In particular, the conjugate moiety of the conjugate as identified herein may preferably a conjugate moiety containing at least one polyalkylene glycol unit of the formula:

X₁-[(CHR₁₁)ₓ-O]ₙ-(Z)_{y}-

where
X₁ is hydrogen or a hydrocarbon which may contain heteroatom(s);e.g C1-C6 alkoxy group
Z is a divalent linkage group, such as C=O or CHR₁₁;
R₁₁ is independently any one of hydrogen, OH, OR₁₂ or CO-R₁₃;
R₁₂ is independently any one of hydrogen or straight or branched C₁-C₆ alkyl chain;
R₁₃ is independently any one of hydrogen, OH, OR₁₂ or NR₁₄R₁₅;
R₁₄ and R₁₅ are independently any one of hydrogen or hydrocarbon which may contain heteroatom(s) and which may form a ring;
n is an integer of 1 to 100;
x is independently an integer of 1 to 10;
y is an integer of 0 to 10.
n may preferably be an integer of 2 to 50, like 2 to 10, in particular 3 to 5.
x may preferably be an integer of 2, 3, or 4, in particular, 2.
y may preferably be an integer of 1 to 5, in particular, 1 to 3, in another preferred embodiment, y may preferably be 0.
X₁ may be preferentially OR₁₆, N(R₁₆)₂, SR₁₆ or COOR₁₆, wherein each R₁₆ is individually hydrogen, benzyl or straight or branched C₁-C₆ alkyl chain. Preferably X1 is a C₁-C₆ straight or branched alkoxy group, like a methoxy, ethoxy or propoxy group.
R₁₁ may be preferably a hydrogen atom.

Thus, the polyalkylene glycol unit mentioned above may preferably contain subunits -[(CHR₁₁)ₓ₋O]ₙ of ethylene glycol, propylene glycol or butylene glycol or combinations thereof. The chain length of each of the polyalkylene glycol units may be in the range of 1 to 100 subunits, preferably, 2 to 50 subunits, like 2 to 10 subunits, particularly in the range of 3 to 5 subunits.

Particularly preferred the conjugate moiety may be a methoxypolyalkyleneglycol-carbonyl-residue wherein the alkylene moiety is an ethylene or propylene moiety.

Hence, preferably the conjugates may be in a pegylated form to increase the solubility in hydrophilic solvents and hydrophilic environment. Furthermore, the conjugate moiety allows protecting the compound moiety, i.e. the active mucosal adjuvant moiety, against enzymatic degradation, structural modification due to change of the pH, mechanical removal, etc. Thus, primarily the stability of the compound is increased. Another beneficial effect of conjugation is to increase the retention time in the individual, e.g. to delay the renal excretion, while being well-tolerated, e.g. being non immunogenic, by said organism. Further, the conjugates, in particular pegylated conjugates improve the bioavailability of the compounds and allow to reduce the dosage administered to the individual.

Specifically, the conjugate moiety may comprise at least two chains having polyalkylene glycol units. That is, the conjugate may be a branched compound wherein each arm contains a polyalkylene glycol unit. Particularly preferred are conjugate moieties wherein the polyalkylene glycol unit is a polyethylene, polypropylene or polybutylene glycol unit.

In a particularly preferred embodiment, the compound moiety according to formula (I) may preferably be covalently linked with the conjugate moiety being a branched moiety wherein at least two arms containing polyethylene glycol units having 3 to 5 ethylene glycol subunits and a methoxy group at the free end of the polyethylene group. In particular, the branched moiety comprises 4 or 6 arms each having 3 ethylene glycol subunits and a methoxy group at the free end of the polyethylene group.

In particular, the conjugate is characterized in that the conjugate moiety is 4armPEG ((S)-10-Amino-6,9,13,16-tetraoxo-N,N',8,14-tetrakis(3,6,9,12-tetraoxatridec-1-yl)-5,8,14,17-tetraazahenicosane-1,21-diamide), 6armPEG or 8armPEG, see also WO 2004/108634 A2.

The conjugate moiety may comprise a polyalkylene glycol unit is a linking group linking two or more of the cyclic dinucleotide compounds according to the present invention. Preferably, the polyalkylene glycol unit is a polyethylene unit containing 2 to 20 ethylene glycol subunits, e.g. 4, 6, 8, 10 or 12 subunits.

The compounds of formula (I) or conjugates thereof may be in the form of pharmaceutically acceptable non-toxic salts thereof. Salts of formula (I) include acid added salts, such as salts with inorganic acids (e.g. hydrochloric acid, sulphuric acid, nitric acid and phosphoric acid) or with organic acids (e.g. acetic acid, propionic acid, maleic acid, olec acid, palmitic acid, citric acid, succinic acid, tartaric acid, fumaric acid, glutamic acid, panthothenic acid, laurylsulfonic acid, methanesulfonic acid and phthalic acid).

Further, the compound for use according to the present invention being a compound according to formula (I) wherein the purine residue is an adenine, xanthine, or hypoxanthine residue or combinations thereof, in particular, both purine residues being adenine.

Moreover, in an embodiment of the present invention, the compound is a compound of formula (I) wherein R₃ is a OH-group, X is an oxygen atom, and Y, Y', Z and Z' are oxygen.

It is particularly preferred that the compound useful according to the present invention is a compound a formula (I) being a cyclic bis(3'-5')diadenylic acid (cyclic di-AMP), c-di-IMP, or c-IAMP, or a cyclic di-AMP thiophosphate, c-di-IMP thiophosphate and c-IAMP thiophosphate or a salt or a solvate thereof, or its conjugates, or combinations of said compounds.

In another aspect, the present invention relates to a pharmaceutical composition comprising a compound as defined herein of formula (I) as an adjuvant for use according to the present invention, a pharmaceutical active ingredient and, optionally, a pharmaceutically acceptable carrier, diluent, preservative, adjuvants, other than the compound of formula (I) as defined herein, immunomodulators or excipients. For example, the active ingredient is an active vaccination component comprising at least one or more different antigens in the form of peptides, proteins, polysaccharides, glycolipids or DNA encoding them or bacterial ghosts, virosomes, or attenuated vaccines, or mixtures thereof.

Preferentially, the antigen(s) are tumor antigen(s) or antigen(s) derived from infectious agents. The infectious agents include those agents which normally enters individual's organism by crossing the mucous membrane.

The pharmaceutical composition comprising adjuvant(s) according to the present invention, an active vaccination component, optionally additional carrier, diluent, preservative, adjuvant other than the adjuvant according to the present invention, immunomodulator or excipient may additionally contain components, like compounds like one or more anti-inflammatory molecules, anti-angiogenic molecules, cytotoxic molecules, immunomodulatory molecules, preferably chemokines, cytokines, CD40 ligand, costimulatory molecules or antibodies or mixtures thereof.

However, the compounds according to formula (I), their conjugates salts and solvates thereof as defined herein for the use as adjuvants may also be a component of a pharmaceutical composition provided in a formulation suitable for parenteral administration, in particular, in subcutaneous, intravenous, intradermal or intramuscular administration.

Further, the compounds and conjugates according to the present invention are useful in tumor therapy including the *in vitro* generation or *in vitro* priming of autologous cells for adoptive cell transfer in tumor therapy and transplantation. Moreover, the adjuvants are useful for the induction of cross-tolerance against microbial components, like endotoxins, to protect against septic shock or other severe forms of diseases induced by microbial components.

The pharmaceutical composition may be administered with a physiologically acceptable carrier to a patient, as described herein. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). The pharmaceutical should suit the mode of administration.

Typically, pharmaceutically or therapeutically acceptable carrier is a carrier medium which does not interfere with the effectiveness of the biological activity of the active ingredients and which is not toxic to the host or patient.

In another preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in a unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

"Therapeutically- or pharmaceutically-effective amount" as applied to the compositions of the instant invention refers to the amount of composition sufficient to induce a desired biological result. That result can be alleviation of the signs, symptoms, or causes of a disease, or any other desired alteration of a biological system. In the present invention, the result will typically involve an increase in the immunological responses to infection or a suppression of the responses to inflammatory processes.

In vitro assays may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The term "administered" means administration of a therapeutically effective dose of the aforementioned pharmaceutical composition to an individual. By "therapeutically effective amount" is meant a dose that produces the effects for which it is administered. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

Further, the pharmaceutical composition may contain additionally components, e.g. compounds like one or more anti-inflammatory molecules, anti-angiogenic molecules, cytotoxic molecules, immunomodulatory molecules, preferably chemokines, cytokines, CD40 ligand, costimulatory molecules or antibodies or mixtures thereof.

In addition, the pharmaceutical composition described herein may be characterized in that the components of the pharmaceutical composition are associated and/or incorporated and/or coated to a physical particle, preferably microparticle, nanoparticle, liposome, ISCOM, copolymer and/or biological particle, preferably bacterial ghosts.

The pharmaceuticals according to the present invention or the compounds for use according to the present invention may be used in methods applicable to both human therapy and veterinary applications, in particular, for human application. The compounds for the use as described herein having the desired therapeutic activity may be administered in a physiologically acceptable carrier to a patient, as described herein. Depending upon the manner of introduction, the compounds may be formulated in a variety of ways as discussed below. The concentration of therapeutically active compound in the formulation may vary from about 0.1-100 wt%. The agents as adjuvant(s) for use in therapeutic or prophylactic vaccination may be administered alone or in combination with other treatments.

The administration of the pharmaceutical composition can be done in a variety of ways as discussed above, including, but not limited to, orally, subcutaneously, intravenously, intra-arterial, intranodal, intramedullary, intrathecal, intraventricular, intranasally, conjunctival, intrabronchial, transdermally, intrarectally, intraperitoneally, intramuscularly, intrapulmonary, vaginally, rectally, or intraocularly. In some instances, for example, in the treatment of wounds and inflammation, the pharmaceutically effective agent may be directly applied as a solution dry spray.

The attending physician and clinical factors will determine the dosage regimen. A typical dose can be, for example, in the range of 0.001 to 1000 µg; however, doses below or above this exemplary range are envisioned, especially considering the aforementioned factors.

In still another aspect, the present invention relates to the use of the compound(s). or salts or solvates thereof as defined herein in a pharmaceutical preparation to control fertility in human or animal populations.

Finally, the present invention relates to kits containing the compound according to the present invention or salts or solvates thereof as an adjuvant and an antigen comprising an antigenic structure and, optionally, a pharmaceutically acceptable carrier, diluent, preservative, adjuvants other than the conjugates according to the present invention, immunomodulators or excipient and instructions for preparing a vaccine.

In an embodiment of the present invention, the pharmaceutical composition for use as defined herein is a pharmaceutical composition for use as defined herein is a pharmaceutical composition for use as a vaccine for neonates and infants.

In an embodiment, the active ingredient is an antigen and the antigen are tumor antigens or antigens derived from infectious agent to prevent or treat infectious diseases, septic shock, cancer, tumors, autoimmune diseases, allergies, or chronic or acute inflammatory processes.

In another embodiment, the pharmaceutical composition is a pharmaceutical composition for use as defined herein wherein said use in a method further comprise the administration of one or more vaccines to the subject when the vaccines comprise one or more antigens selected to stimulate and an immune response to the pathogen expressing one or more of the antigens.

The pharmaceutical composition according to the present invention may be a vaccine comprising inactivated or attenuated bacteria or viruses comprising the antigen of interest, purified antigens, live viral or bacterial delivery vectors recombinantly engineered to express and/or secrete the antigens, antigen presenting cell (APC) vectors comprising cells that are loaded with the antigens or transfected with a composition comprising a nucleic acid encoding the antigens, liposomal antigen delivery vehicles, or naked nucleic acid vectors encoding the antigens.

The pharmaceutical composition according to the present invention is in an embodiment a combined composition for simultaneous, separate or sequential use in preventing or treating infectious diseases, cancers, tumors, autoimmune diseases or allergies or chronic or acute inflammatory processes or to control fertility in human or animal populations.

Further, the present invention relates to a kit comprising the compound of formula (I) as defined herein as an adjuvant, an antigen or antigenic composition comprising antigenic structure as active ingredient, in particular, as active vaccination component, and, optionally, a pharmaceutically acceptable carrier, diluent, preservative, adjuvants other than the compound of general formula (I) as defined herein, immunomodulators or excipients.

Finally, the present invention describes a method of prophylactically or therapeutically treating unborn children, neonates and infants by vaccination. Said method comprises the step of administering a compound according to formula I, in particular, CDA, to an unborn child neonate or infant as adjuvant in the context of treating said unborn children, neonates or infants with an active ingredient, in particular, with an active vaccination component for prophylactically or therapeutically treating the unborn child, neonate or infant, in particular, for vaccination purposes.

The skilled person is well aware of suitable dosages and ways of administrating said components.

As noted, the method may encompass treating the unborn in the pregnant women. The skilled person is aware of suitable rules of administration and dosages accordingly.

These and other embodiments are disclosed and encompassed by the description and examples of the present invention. The present invention will be described further by the way of examples without limiting the same.

### Examples

### METHODS

### Animals

WT C57BL/6 and *Ifnar1-*/*-* mice were bred at the Helmholtz Centre for Infection Research (HZI). All animals were on the C57BL/6 background and were kept under specific pathogen-free conditions. All experiments were performed in compliance with the German animal protection law (TierSchG BGBI. I S 1105; 25.05.1998) and were approved by the Lower Saxony Committee on the Ethics of Animal Experiments as well as the responsible state office (Lower Saxony State Office of Consumer Protection and Food Safety), under permit numbers 33.11.42502-04-105/07 and 33.4-42502-04-13/1281.

### Human adult and cord blood

Adult peripheral blood for controls was collected from healthy volunteers. Cord blood was obtained by needle puncture of the umbilical cord vein right after delivery of healthy newborn donors at the Women's Clinic (Braunschweig Central Hospital). Signed informed consent from parents was obtained, ensuring that no ulterior modification of the genetic material contained or therapeutic use was involved in our study. Citrate anti-coagulated cord blood bags (Macopharma, France) were stored at room temperature before the *in vitro* assays.

### In vitro assays using human blood

Adult or cord blood was mixed 1:1 with 37°C warm RPMI 1640 medium (Gibco) and incubated with adjuvants and controls according to previously described method (Dowling, D. J., et. al., 2013, PLoS One 8 (3):e58164.). Briefly, 20 µl of the adjuvants 10X were added to 180 µl of diluted blood in quadruplicates (in a 96 U shape well plate), and incubated at 37°C, 5% CO₂ for 6 h. Supernatant fluids were assayed by flow cytometry in multiplex arrays (Biolegend) or in an ELISA reader for IL-1β (eBioscience).

### Immunizations

We assessed the capability of CDA to promote antigen-specific immune responses in neonates following mucosal or subcutaneous vaccination. To this end, mice were immunized by s.c. or i.n. route in a prime/boost regime (two boost doses) with a two-week-window interval between vaccinations. Neonates (7-9 days old) were vaccinated by i.n. inoculation (6 µl) of CDA+OVA, R848+OVA, OVA alone or control buffer. Alternatively, the neonatal dose was skipped and a prime was given at young age (21-23 days old) in order to determine the usefulness of the neonatal dose. In a different setting for testing B cell activation, neonatal mice were immunized once and sampled 14 days after vaccination. Mice received a dose consisting of 20 µg OVA (EndoGrade, Hyglos GmbH, Germany) and 7.5 µg of CDA (InvivoGen, France). Controls received PBS (Hyglos, Germany). After a 2-3 week interval, vaccinated mice were tested for T cell responses or underwent pathogen challenge (n=3-5 per group, experiments always done twice).

### Lymphocyte isolation, intracellular cytokine staining

Spleens and LN were homogenized by mechanical disruption on a cell strainer. Red blood cells were lysed by ACK buffer. Lymphocytes were washed with PBS and counted for subsequent subpopulation isolation by positive selection on LS columns (Miltenyi, Germany) or *in vitro* re-stimulation. For intracellular cytokine staining, lymphocytes were incubated overnight in the presence of antigen (OVA, 20 µg/ml) or media (control). During the last 6 h of antigenic stimulation, cells were treated with brefeldin (5 µg/ml) and monensin (6 µg/ml). Cells were stained with antibodies specific for surface receptors and live/dead cell marker for 30 min at 4°C (see reagents below). Cells were then washed in PBS, fixed in 1% paraformaldehyde and permeabilized with 0.5% saponin and 0.5% bovine serum albumin (BSA) in PBS for 1 h at 4°C. Intracellular staining with cytokine specific antibodies (see reagents) was performed in permeabilization solution for 45 min at 4°C.

### Viral challenge

Under anesthesia (isoflurane), vaccinated animals and controls received i.n. 10⁵ PFU of the H1N1 PR8 influenza A/WSN/33 (WSN)-OVA(I) strain, which expresses the MHC class I-restricted SIINFEKL immunodominant peptide from OVA within the hemagglutinin (Topham, D. J., et. al., 2001, J Immunol 167 (12):6983-90). Body weight and health parameters (e.g., piloerection and motility) were monitored daily during the first 12-15 days post infection.

### Data processing

Flow cytometry data were acquired on a LSR Fortessa with FACSDiva software (BD Biosciences, USA) and were analyzed with FlowJo software (FlowJo, LLC, USA). Other data were analyzed with Microsoft Excel and GraphPad Prism 5 statistical software (GraphPad Software Inc., USA). All observed differences were tested for statistical significance at p<0.05 by unpaired Student's *t*-test using GraphPad Prism 5.

### Reagents

BSA was purchased from Roth (Karlsruhe, Germany), saponin from Serva (Heidelberg, Germany) and live-dead UV-blue staining and CFSE were obtained from Molecular Probes (Eugene, Oregon). Anti-mouse antibodies: CD3 (clone 500A2, V500 conjugated) and anti-IL-2 (clone JES6-5H4, APC-Cy7 conjugated) were obtained from BD Bioscience (USA); anti-CD4 (clone RM4-5, PE-Cy7 conjugated), anti-TNF-a (clone MPG-XT22, PerCP-eF710 conjugated), anti-IL-4 (clone 11B11, APC conjugated) were purchased from eBioscience Inc. (USA); and anti-CD8 (clone 53-6.7, BV650 conjugated), anti-IFN-γ (clone XMG1.2, BV711 and BV785 conjugated) were obtained from Biolegend (USA). IL-1β ELISA kit was purchased from Affymetrix (eBioscience). Multiple human cytokine detection kit, Human Th Cytokine Panel LEGENDplex was purchased from Biolegend. R848 and CDA were purchased from InvivoGen.

### Statistical analysis

The statistical significance of the differences observed between the different experimental groups was analyzed using Student's *t*-test (GraphPad Prism). Differences were considered significant at p < 0.05.

### RESULTS

### CDA stimulates a Th1 cytokine profile on cells derived from human cord blood.

One of the well-established methods to assess adjuvant immunogenicity in human neonates is the use of cord blood as a surrogate (Dowling, D. J., et. al., 2013, PLoS One 8 (3):e58164. doi:). Therefore, to assess the secretory cytokine profile elicited by CDA in neonates, we incubated cord and adult blood in the presence of adjuvants and controls for 6 h. A bead-based multiplexed immunoassay array was employed in order to detect cytokines in the supernatant. We found that TNF-α and IFN-γ (cytokines produced by T helper 1 -Th1- cells) were present at higher concentrations when the cord blood was treated with CDA as compared to R848 or medium. Nevertheless, these differences were not observed when testing blood from adults. We also detected higher concentrations of IL-6, IL-21 and IL-10 (cytokines belonging to the T follicular helper -Tfh- differentiation profile). Slightly similar results were obtained when testing blood from adults. Interestingly, the stimulation by CDA of cytokines produced by T helper 2 and 17 (Th2 and Th17) cells was more modest compared to what was observed after R848 treatment and in control cells from both adult and cord blood. Th17 cytokines were particularly decreased after treatment with CDA, and also IL-9 was slightly decreased in cord blood samples and this difference was accentuated over the other treatments in adult blood. Remarkably, CDA increased the secretion of Th1 cytokines, which are important to counteract the Th2 bias present at birth (Debock, I., et. al., 2013, J Immunol 191 (3):1231-9. doi: 10.4049/jimmunol.1203288). Interestingly, by ELISA we found that pro-inflammatory cytokines, like IL-1β, where slightly decreased in CDA-treated blood with respect to R848, in adult as well as in cord blood.

### CDA-adjuvanted neonatal dose elicits multiple-cytokine producers among antigen-specific T cells.

The above-mentioned *in vitro* approach is very useful as an initial screening for the evaluation of cytokine profiles promoted by candidate adjuvants after stimulation of different types of cells. However, it does not allow accurate prediction of important effector functions of an adjuvant, such as CTL stimulation capabilities, target cell subpopulations and capacity to confer protective immunity. Therefore, neonatal mice were immunized to assess the *in vivo* performance of CDA in an active vaccination setting. To this end, a group of mice received a neonatal prime at day 7 after birth (day 0 of the vaccination schedule) followed by two boosts on days 14 and 28 of the vaccination schedule, a second group received the prime at day 21 after birth (young age, day 14 of the vaccination schedule) followed by one boost on day 28, and a third group of mice received the prime at adult age (more than 8 weeks) followed by two boosts 14 and 28 days later (Fig. 1A). To assess vaccination effectivity, the frequencies of cytokine producers among the CD4⁺ and CD8⁺ T cell populations were evaluated by intracellular cytokine staining and flow cytometry. The production of multiple cytokines (especially IL-2, IFN-γ, and TNF-α) by T cells has been described to correlate with vaccine protective efficacy (Darrah, P. A., et. al., 2007, Nat Med 13 (7):843-50. doi: 10.1038/nm1592). Thus, the frequency of T cells producing single cytokines or combinations (positive events/million) was analyzed by flow cytometry (Fig. 1B, C). The same vaccination schedule was used for groups receiving R848 as "gold standard" adjuvant that has been extensively used to promote neonatal immunity (Zhang, X., N. et. al., 2014, J Infect Dis 210 (3):424-34. doi: 10.1093/infdis/jiu103). Although R848 has been claimed to elicit potent immunity when used in neonates (Holbrook, B. C., et. al., 2017, Immunology. doi: 10.1111/imm.12845), we found that in our experimental setting the use of R848 did not significantly increase the frequency of multifunctional T cells expressing IFN-γ, TNF-α and IL-2. Moreover, the presence of CD4⁺ T cells that produce any possible combination of the four analyzed cytokines (IFN-γ, TNF-α, IL-17 and IL-2) was indeed reduced when a neonatal dose of R848 was used as compared to the treatment where this dose was skipped or in adult controls (Fig. 1A). In contrast, the use of a neonatal dose of CDA-adjuvanted vaccine increased the frequency of triple, double and single cytokine producers, when compared with the group where the neonatal dose was skipped. Interestingly, neonatal priming resulted in better stimulation of multifunctional T cells than priming at a later age when CDA was used as adjuvant (Fig. 1B, upper part). A subcutaneous (s.c.) vaccination at days 6-9 with ovalbumin (OVA) + CDA or Alum also resulted in an increase of triple cytokine producers among CD4⁺ T cells from the CDA+OVA vaccinated animals over the other treatments.

In order to characterize more accurately the neonatal cellular immune response, the production of intracellular cytokines was measured in CD8⁺ T cells. Multifunctional CD8+ cells were only stimulated when mice received a neonatal priming with OVA+CDA (Fig. 1C upper left). When animals were vaccinated as adults the responses were stronger for any particular cytokine or its combinations (Fig. 2C, upper right). In contrast, when R848 was used as neonatal adjuvant, multifunctional CD8⁺T cells were reduced as compared to the group where the neonatal dose was skipped. In a similar fashion as for the groups receiving CDA as adjuvant, when mice were vaccinated as adults the responses were stronger for any considered cytokine combination (Fig. 1C, lower part). It is important to note that when the capacity of neonatal priming to generate multifunctional CD8⁺ T cells producing IFN-γ, TNF-α and IL-2 was assessed, although both adjuvants promoted similar immune responses in adults, only CDA increased the frequency of triple cytokine producers when administered at neonatal age (Fig. 1B, C).

### A CDA-adjuvanted neonatal vaccine induces B cell activation and maturation.

A key feature to determine vaccine efficacy is also the activation of B cells. Importantly, neonatal B cells are regulated by type I interferon (IFN-α/β) on their capacity to moderate inflammation upon infection via IL-10 (Zhang, X., et. al., 2007, J Exp Med 204 (5):1107-18. doi: 10.1084/jem.20062013). It has also been reported that IFN-α/β is necessary for the activation and development of B cells in neonates (Jans, J., et. al., 2017, J Allergy Clin Immunol 139 (6):1997-2000.e4. doi: 10.1016/j.jaci.2016.10.032). In order to assess whether CDA-mediated generation of IFN-α/β was necessary for B cells maturation after vaccination, we vaccinated WT and IFNAR1-/- neonatal mice with CDA+OVA, OVA alone or a vehicle control. Neonatal mice were vaccinated by i.n. route at days 7-9 after birth, and euthanized 14 days after vaccination to measure the development and activation of B lymphocytes after *in vitro* re-stimulation with OVA. We observed that the number of viable B cells after antigen-specific re-stimulation of splenocytes was significantly higher in WT mice vaccinated with CDA+OVA than in all other treatment groups (data not shown). Importantly, the expression of the activation marker CD69 and the surface maturation marker CD86 on the B cell subset was enhanced in WT mice vaccinated with CDA+OVA when compared with the other groups (Fig. 2A, B). Although differences were not statistically significant in neonates, the trend was maintained and acquired significance in adults after a prime and boost vaccination schedule. This suggests that neonatal vaccination with CDA triggers IFN-α/β dependent mechanisms that are necessary for the activation and development of B cells.

### Neonatal CDA vaccination promotes a protective immune response in adult mice.

In order to assess the biological significance of our findings, neonatal mice were vaccinated using CDA as adjuvant, controls received R848 as control neonatal adjuvant (Levy, O., et. al., 2004, J Immunol 173 (7):4627-34). Mice were vaccinated according to the scheme displayed in Figure 2A, which consisted of a prime immunization given at day 7 of life (day 0 of the vaccination schedule) followed by two boosts on days 14 and 28 of the vaccination schedule. After vaccination, mice were challenged with a recombinant OVA-peptide expressing H1N1 influenza virus (Topham, see above) and mice were monitored for 14 days after infection. In order to determine if the humoral response is stronger upon neonatal vaccination with CDA, OVA-specific IgG titers were measured in mice sera before H1N1 challenge. Neonatal vaccination with CDA+OVA elicited the highest titers of antigen-specific IgG, being significantly higher not only in comparison to the OVA alone and PBS controls, but also in respect to the titers observed in mice vaccinated using R848 (Fig. 3A). Noteworthy, 1 mouse per group died in the control group and in the R848 group after challenge; whereas no deaths were observed in the OVA only or in the CDA+OVA group. Mice vaccinated with CDA+OVA maintained and gained weight over the 14 days after challenge (Fig. 3B). In contrast, mice vaccinated with the TLR7-8 agonist R848, OVA alone or PBS lost weight during the treatment, particularly between day 7 and 9 post challenge (Fig. 3B, C). The protection conferred by CDA was also significantly higher than the one conferred by the CDA-based vaccination where the neonatal dose was skipped (data not shown). The differences in protection between CDA and the other adjuvants and controls were significant during the 48 h of maximal weight loss (Fig. 3C).

### DISCUSSION

Due to the prevalence of upper airway infections during the first year of life, and the trauma associated with needle vaccination, i.n. delivery of vaccines is an attractive immunization strategy. Therefore, we use a mucosal adjuvant administered i.n., whose vaccination potency is not restricted to this administration route (sub-cutaneous vaccination was also performed, where our adjuvant showed superiority to alum salts). We had previously shown that CDA is a potent naturally occurring adjuvant that is able to elicit strong immune activation, leading to not only humoral but also cellular responses (Ebensen, T., et. al., 2011, Vaccine 29 (32):5210-20. doi: 10.1016/j.vaccine.2011.05.02610.1016/j.vaccine.2011.05.026. Epub 2011 May 25). The cellular response also encompasses the induction of a CTL component, whose presence and efficacy depends on the stimulation of IFN-α/β, and its cross priming capabilities (Lirussi, D., et. al., 2017, EBioMedicine 22:100-111. doi: 10.1016/j.ebiom.2017.07.016). Thus, one of the main advantages of the CDA over most vaccine adjuvants is its capability to generate humoral antibody responses as well as cellular CTL responses. In this regard, neonatal APC were shown to be competent in MHC class I antigenic processing and presentation, as well as in further stimulation of CTL responses. In order to determine which cytokine profile is promoted by CDA vaccination; we assessed the cytokine production pattern elicited after CDA stimulation of human adult blood and cord blood (as a neonate surrogate) *in vitro.* We observed that CDA induces higher concentrations of Th1 and Tfh cytokines than controls and R848. These findings were further confirmed *in vivo,* by using a murine model. Since first dose effectiveness is a controversial issue on infant vaccination(Carazo Perez et al. 2017, see above), we measured the differences in cytokine secretion when the neonatal dose was skipped *in vivo.* The obtained results demonstrated that while a neonatal dose containing the "gold standard" adjuvant R848 has negative effects on multiple cytokine producers, a neonatal dose adjuvanted with CDA has beneficial effects as a prime. Moreover, we were able to corroborate that the cytokine profiles detected in CDA-treated human cord blood were maintained *in vivo* for multiple cytokine producers. This effect is an advantageous feature of CDN over TLR agonists, since the former has its signaling pathways and molecular targets conserved between mice and humans, whereas TLR agonists do not. We demonstrated here not only that these properties are conserved at early ages (neonatal period), but also that CDA performs better than other proposed neonatal adjuvants. When evaluating the humoral response we showed that neonatal vaccination with CDA elicits higher titers of antigen-specific IgG than those observed using R848. In agreement with this enhanced antibody production, we demonstrated that CDA promotes the activation of B cells at neonatal and adult ages, earlier upon vaccination. The activation of B cells by CDA was also dependent on type I IFN signaling. Besides R848, other TLR agonists like MPLA and CpG showed promising results as neonatal adjuvants. Neonatal vaccination with CDA+OVA promoted higher antigen-specific IgG titers and protection against challenge than what observed in mice receiving MPLA or CpG as adjuvants (data not shown).

It has been shown that although most of the adaptive immunity is deficient in newborns, CTL responses are normally developed at the neonatal age. Therefore, adjuvants that promote the generation of CTL responses are highly desirable for neonatal vaccination. The results presented here showed that CDA is able to promote a protective CTL response capable of protecting against a genetically modified influenza virus that expresses a CD8 restricted epitope, and that this CTL response is dependent on the administration of the neonatal prime dose. In our experimental system, the neonatal administration of the TLR 7/8 agonist R848 as adjuvant resulted in a detrimental effect post immunization. In contrast, the effect of CDA-mediated immune stimulation triggers protective immunity. Altogether, our data support a promising role of CDA as a neonatal adjuvant, which can be exploited as a tool in vaccines for newborns and infants in order to reduce antigen load or the number of doses required to achieve protective immunity.

## Claims

1. A compound according to formula (I) 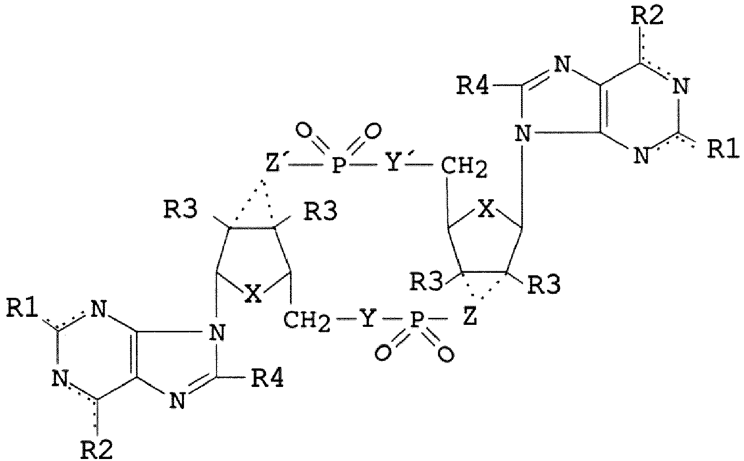 wherein
X is independently from one another S, N, O, CH₂;
Y, Y' is independently from one another NH, CH₂, O;
Z, Z' is independently from one another NH, CH₂, O;
R₁ is independently from one another hydrogen or O or absent;
R₂ is independently from one another NH₂, O, H, or a hydrogen;
R₃ is independently from one another absent if a covalent bond is present between the Z or Z' and the C atom, or is hydrogen, OH, halogen, a straight or branched C₁-C₆ alkyl group, or a straight or branched C₁-C₆ alkoxy group which may optionally be substituted;
R₄ is independently from one another hydrogen, halogen, or a straight or branched C₁-C₆ alkyl group which may optionally be substituted;
... is a single or double bond;
or conjugates thereof, and salts or solvates thereof, for use in a method of inducing or promoting an immune response in an individual wherein said individual is an unborn child, a neonate or an infant.

2. The compound according to claim 1 for use as an adjuvant in the prophylactic or therapeutic treatment of a disease, in particular, for use in therapeutic or prophylactic vaccination.

3. The compound according to any one of the preceding claims i) for use as a mucosal adjuvant, in particular, for intranasal, intra NALT, oral, intra-rectal, intrafollicular, transfollicular, intrapulmonary, intrabronchial, intrathecal, conjunctival, intra-vaginal or intra-urethral administration, administration into the milk ducts of the breast or by inhalation or ii) for use for parenteral administration, in particular, for subcutaneous, intravenous, intradermal, intrafollicular or intramuscular administration.

4. The compound for use according to any one of the preceding claims, wherein the compound is a conjugate of a compound of formula (I) with a conjugate moiety which is a water-soluble and physiologically tolerated polymer, in particular, wherein the conjugate moiety contains at least one polyalkylene glycol units of the formula:
X₁-[(CHR₁₁)ₓ-O]ₙ-(Z)_{y}-
where
X₁ is hydrogen or a hydrocarbon which may contain heteroatom(s);
Z is a divalent linkage group, such as C=O or CHR₁₁;
R₁₁ is independently any one of hydrogen, OH, OR₁₂ or CO-R₁₃;
R₁₂ is independently any one of hydrogen or straight or branched C₁-C₆ alkyl;
R₁₃ is independently any one of hydrogen, OH, OR₁₂ or NR₁₄R₁₅;
R₁₄ and R₁₅ are independently any one of hydrogen or hydrocarbon which may contain heteroatom(s) and which may form a ring;
n is an integer of 1 to 100;
x is independently an integer of 1 to 10;
y is an integer of 0 to 10.

5. The compound for use according to claim 4 **characterized in that** the conjugate moiety comprises at least two chains having polyalkylene glycol units, in particular, the polyalkylene glycol units in the conjugate moiety are polyethylene units, polypropylene units and/or polybutylene units.

6. The compound for use according to claims 4 or 5 **characterized in that** the conjugate moiety is a methoxypolyethylenglykol-carbonyl residue.

7. The compound for use according to any one of the preceding claims, **characterized in that** in the compound according to formula (I) any one of the purine residue is an adenine, xanthine or hypoxanthine residue or combinations thereof, in particular, both purine residues being adenine.

8. The compound for use according to any one of the preceding claims **characterized in that** in formula (I) R₃ is a OH group, X is an oxygen atom, and Y, Y', Z and Z' are oxygen.

9. The compound for use according to any one of the preceding claims **characterized in that** the compound according to formula (I) is a cyclic bis(3'-5')diadenylic acid (CDA), c-diIMP, c-IAMP, or a cyclic di-AMP thiophosphate, c-di-IMP thiophosphate, and c-AMP-IMP thiophosphate or a salt or a solvate thereof, or its conjugates, or combinations of said compounds.

10. A pharmaceutical composition comprising a compound as defined in any one of claims 1 to 9 as an adjuvant for use according to any one of claims 1 to 9, a pharmaceutical active ingredient, and, optionally a pharmaceutically acceptable carrier, diluent, preservative, adjuvants, other than the compound as defined in any one of claims 1 to 9, immunomodulators or excipient, in particular, being a vaccine for unborn children, neonates and infants.

11. The pharmaceutical composition for use according to claims 9 or 10 wherein the active ingredient is an antigen and the antigen are tumour antigens or antigens derived from infectious agent to prevent or treat infectious diseases, septic shock, cancer, tumours, autoimmune diseases, allergies, or chronic or acute inflammatory processes.

12. The pharmaceutical composition for use according to any one of claims to 11 wherein said use in a method further comprise administration of one or more vaccines to the subject wherein the vaccines comprise one or more antigens selected to stimulate an immune response to the pathogen expressing one or more of the antigens.

13. The pharmaceutical composition according to any one of claims 9 to 12 wherein the vaccine comprise inactivated or attenuated bacteria or viruses comprising the antigen of interest, purified antigens, live viral or bacterial delivery vectors recombinantly engineered to express and/or secrete the antigens, antigen presenting cell (APC) vectors comprising cells that are loaded with the antigens or transfected with a composition comprising a nucleic acid encoding the antigens, liposomal antigen delivery vehicles, or naked nucleic acid vectors encoding the antigens.

14. A pharmaceutical composition according to any one of claims 9 to 13 as a combined composition for simultaneous, separate or sequential use in preventing or treating infectious diseases, cancers, tumours, autoimmune diseases or allergies, or chronic or acute inflammatory processes or to control fertility in human or animal populations.

15. A kit comprising a compound according to any one of claims 1 to 8 as an adjuvant, an antigen comprising antigenic structure as active vaccination component and, optionally, a pharmaceutically acceptable carrier, diluent, preservative, adjuvants other than the compound as defined in any one of claims 1 to 8, immunomodulators or excipient.
